# EUROPEAN PATENT SPECIFICATION

(11) **EP 2 979 728 B1**
(45) Date of publication and mention of the grant of the patent: **21.02.2018**
(21) Application number: 14773040.2
(22) Date of filing: 22.01.2014
(51) Int. Cl.: A61N 5/10

(54) **NEUTRON CAPTURE THERAPY DEVICE**
THERAPIEVORRICHTUNG MIT NEUTRONENERFASSUNG
DISPOSITIF DE THÉRAPIE PAR CAPTURE NEUTRONIQUE

(30) Priority: 29.03.2013 JP 2013071763
(43) Date of publication of application: 03.02.2016
(73) Proprietor: Sumitomo Heavy Industries, Ltd., Tokyo 141-6025 (JP)
(72) Inventor: TAKI, Kazuya, Yokosuka-shi Kanagawa 237-8555 (JP)
(74) Representative: Louis Pöhlau Lohrentz
(86) International application number: PCT/JP2014/051256
(87) International publication number: WO 2014/156245

(56) References cited:
- WO-A1-2007/093965
- WO-A1-2012/014671
- JP-A- 2013 062 193
- JP-A- 2013 167 467
- BAYANOV B ET AL: "First neutron generation in the BINP accelerator based neutron source", APPLIED RADIATION AND ISOTOPES, ELSEVIER, OXFORD, GB, vol. 67, no. 7-8, 1 July 2009 (2009-07-01) , pages S285-S287, XP026195974, ISSN: 0969-8043, DOI: 10.1016/J.APRADISO.2009.03.077 [retrieved on 2009-03-27]
- TANAKA H ET AL: "Experimental verification of beam characteristics for cyclotron-based epithermal neutron source (C-BENS)", APPLIED RADIATION AND ISOTOPES, vol. 69, no. 12, 21 March 2011 (2011-03-21), pages 1642-1645, XP028332929, ISSN: 0969-8043, DOI: 10.1016/J.APRADISO.2011.03.020 [retrieved on 2011-03-21]
- Yoshinori Sakurai ET AL: "Dose distributions in a human head phantom for neutron capture therapy using moderated neutrons from the 2.5 MeV proton- 7 Li reaction or from fission of 235 U Experimentalverification of improved depth-dose distribution A design study for an accelerator-based epithermal neutron beam for BNCT Dose-d", Phys. Med. Biol. PHYSICS IN MEDICINE AND BIOLOGY Phys. Med. Biol, 1 January 2001 (2001-01-01), pages 2681-2695, XP055327156, Retrieved from the Internet: URL:http://iopscience.iop.org/article/10.1 088/0031-9155/46/10/311/pdf

## Description

### Technical Field

The present invention relates to a neutron capture therapy device.

### Background Art

As an expedient of a radiation therapy in cancer treatment and the like, there is a neutron capture therapy in which tumor treatment is carried out by performing irradiation of a neutron beam. In the neutron capture therapy, a neutron capture element compound having tumor clustering properties is applied to a patient in advance. Thereafter, as a tumor of the patient is irradiated with a neutron beam, neutrons and the neutron capture element compound react to each other, and radial rays are generated, thereby performing tumor treatment.

PTL 1 is a technical literature related to the neutron capture therapy. PTL 1 discloses a neutron beam irradiation apparatus including a neutron beam generation section which generates a neutron beam by irradiating a target with a charged particle beam, and measurement means for measuring a radiation dose of irradiation of the charged particle beam in real time during irradiation of the neutron beam.

### Citation List

### Patent Literature

[PTL 1] Pamphlet of International Publication No. WO 2012/014671

Reference is made also to the publication "First neutron generation in the BINP accelerator based neutron source", B. Bayanov et al, Applied Radiation and Isotopes 67 (2009) S285-S287.

### Summary of Invention

### Technical Problem

According to the above-described apparatus in the related art, improvement of irradiation accuracy of a neutron beam is achieved by measuring a radiation dose of irradiation of a charged particle beam in real time during irradiation of the neutron beam. However, measurement is performed for only the irradiation condition of the charged particle beam before generating the neutron beam, and thus, there is still room for improvement.

Therefore, in the related technical field, it is desired to provide a neutron capture therapy device in which improvement of irradiation accuracy of a neutron beam can be achieved. Solution to Problem

The invention is defined in the appended set of claims. In order to solve the problem described above, according to an aspect of the invention, there is provided a neutron capture therapy device which irradiates an irradiation target with a neutron beam, the apparatus including: an accelerator configured to emit a charged particle beam; a neutron beam generation section configured to include a target which generates the neutron beam by being irradiated with the charged particle beam; current value detection means for detecting a current value of the charged particle beam with which the target is irradiated; and gamma ray dose measurement means for measuring a radiation dose of a gamma ray which is generated from the neutron beam generation section in response to irradiation of the charged particle beam.

In the neutron capture therapy device according to the aspect of the present invention, since the current value of the charged particle beam for irradiating the target is detected, and the radiation dose of the gamma ray generated in the neutron beam generation section in response to irradiation of the charged particle beam is measured, it is possible to detect both the irradiation condition of the charged particle beam before generating a neutron beam and the generation condition of the gamma ray after the neutron beam is generated. Therefore, compared to the case in the related art in which only the irradiation condition of the charged particle beam is detected, irradiation accuracy of the neutron beam can be improved.

The neutron capture therapy device described above further includes neutron beam dose calculation means for calculating a radiation dose of the neutron beam which is generated in the neutron beam generation section, based on a detection result of the current value detection means and a measurement result of the gamma ray dose measurement means.

According to the neutron capture therapy device, since the radiation dose of a neutron beam heading toward a patient can be calculated based on the detection result of the current value detection means and the measurement result of the gamma ray dose measurement means, it is possible to determine whether or not the radiation dose of the neutron beam complies with the treatment plan, thereby being advantageous to improve irradiation accuracy of the neutron beam.

The neutron capture therapy device described above, may further include control means for controlling irradiation of the charged particle beam with respect to the target based on a calculation result of the neutron beam dose calculation means.

According to the neutron capture therapy device, since the irradiation of the charged particle beam with respect to the target is controlled based on the calculation result of the neutron beam calculation means, the irradiation of a neutron beam can be controlled so as to obtain the radiation dose which complies with the treatment plan. Therefore, it is possible to improve the irradiation accuracy of the neutron beam with which a patient is irradiated.

The neutron capture therapy device described above, may further include determination means for determining whether generation of the neutron beam is normal or abnormal, based on the detection result of the current value detection means and the measurement result of the gamma ray dose measurement means.

According to the neutron capture therapy device, since it is determined whether generation of a neutron beam is normal or abnormal based on the detection result of the current value detection means and the measurement result of the gamma ray dose measurement means, it is possible to detect an abnormality which occurs in the generation of the neutron beam such as an excessive radiation dose of the gamma ray compared to the radiation dose of irradiation of the charged particle beam.

In addition, in the neutron capture therapy device described above, the determination means may stop irradiating the target with the charged particle beam when it is determined that generation of the neutron beam is abnormal.

According to the neutron capture therapy device, since the irradiation of the charged particle beam with respect to the target stops when it is determined that generation of a neutron beam is abnormal, it is possible to prevent a patient from being irradiated with the neutron beam at a radiation dose which does not comply with the treatment plan.

In addition, the neutron capture therapy device described above, may further include: a moderator configured to moderate the neutron beam which is generated in the neutron beam generation section; and a beam blockage body configured to be provided so as to surround the moderator, in which the gamma ray dose measurement means may include a gamma ray detection unit which detects the gamma ray, and in which the gamma ray detection unit may be provided on an upstream side of the charged particle beam from the moderator on the periphery of the target.

According to the neutron capture therapy device, since the gamma ray detection unit is provided on the upstream side of the charged particle beam from the moderator on the periphery of the target, it is possible to detect the gamma ray generated from the neutron beam generation section before the gamma ray is blocked by other members, thereby contributing to improvement of detection accuracy of the gamma ray. In addition, maintenance of the gamma ray detection unit is also easily performed.

The neutron capture therapy device described above, may further include: a moderator configured to moderate the neutron beam which is generated in the neutron beam generation section; and a beam blockage body configured to be provided so as to surround the moderator, in which the gamma ray dose measurement means may include a gamma ray detection unit which detects the gamma ray, and in which the gamma ray detection unit may be provided between the moderator and the beam blockage body.

According to the neutron capture therapy device, since the gamma ray detection unit is provided between the moderator in which a neutron beam travels and the beam blockage body which surrounds the moderator, it is possible to capture the gamma ray in the forward direction of the neutron beam, and thus, it is possible to detect a gamma ray which is advantageous to measure the neutron beam. In addition, according to the neutron capture therapy device, since the gamma ray can be prevented from being missed in detection, it is possible to prevent an abnormal state from being missed in detection.

In addition, the neutron capture therapy device described above, may further include: a collimator configured to include an opening for shaping an irradiation field of the neutron beam; and neutron beam dose measurement means for measuring the radiation dose of the neutron beam, in which the neutron beam dose measurement means may include a neutron beam detection section which detects the neutron beam, and in which the neutron beam detection section may be provided inside the collimator and is exposed to the opening.

According to the neutron capture therapy device, since the neutron beam detection section which detects a neutron beam is provided inside the collimator and is exposed to the opening of the collimator through which the neutron beam passes, it is possible to detect the neutron beam heading toward a patient without blocking the traveling path of the neutron beam.

### Advantageous Effects of Invention

In a neutron capture therapy device according to an aspect of the present invention, irradiation accuracy of a neutron beam can be improved.

### Brief Description of Drawings

FIG. 1 is a diagram illustrating a neutron capture therapy device according to a first embodiment.
FIG. 2 a cross-sectional diagram illustrating a neutron beam detection section which is provided inside a collimator.
FIG. 3(a) is a diagram for illustrating abnormality determination conducted based on a relationship between a dose of gamma rays and a dose of protons. FIG. 3(b) is a diagram for illustrating abnormality determination conducted based on a relationship between a dose of neutrons and a dose of protons.
FIG. 4 is a diagram illustrating the neutron capture therapy device according to a second embodiment.

### Description of Embodiments

Hereinafter, favorable embodiments of the present invention will be described in detail with reference to the drawings.

### [First Embodiment]

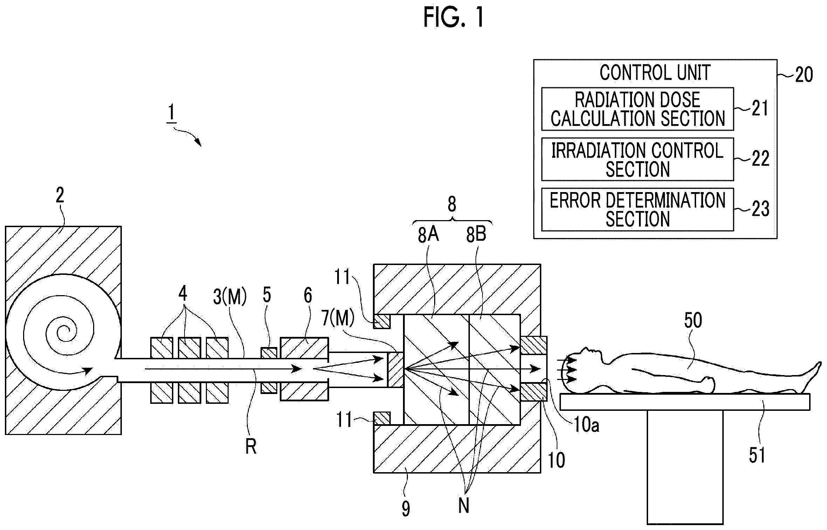

As illustrated in FIG. 1, a neutron capture therapy device 1 according to a first embodiment is an apparatus which is used to perform cancer treatment and the like using the boron neutron capture therapy (BNCT). For example, irradiation of a neutron beam N is performed with respect to a tumor of a patient (an irradiation target) 50 to whom boron (¹⁰B) is applied.

The neutron capture therapy device 1 includes a cyclotron 2. The cyclotron 2 is an accelerator which generates a charged particle beam R by accelerating a charged particle such as an anion. According to the present embodiment, the charged particle beam R is a proton beam which is generated by stripping an electrical charge from an anion. For example, the cyclotron 2 is capable of generating the charged particle beam R of 60 kW (= 30 MeV X 2 mA) having the beam radius of 40 mm. The accelerator may be a synchrotron, a synchrocyclotron, or a linear accelerator without being limited to the cyclotron.

The charged particle beam R emitted from the cyclotron 2 is sent to a neutron beam generation section M. The neutron beam generation section M is configured to include a beam duct 3 and a target 7. The charged particle beam R emitted from the cyclotron 2 passes through the beam duct 3 and travels toward the target 7 which is disposed at an end portion of the beam duct 3. A plurality of quadrupole electromagnets 4, a current monitor (current value detection means) 5, and a scanning electromagnet 6 are provided along the beam duct 3. The plurality of quadrupole electromagnets 4 perform beam axis adjustment of the charged particle beam R by using the electromagnets, for example.

The current monitor 5 detects the current value (that is, an electrical charge, and a radiation dose rate of irradiation) of the charged particle beam R with which the target 7 is irradiated, in real time. A non-destructive DC current transformer (DCCT) capable of measuring a current without affecting the charged particle beam R is used as the current monitor 5. The current monitor 5 outputs a detection result to a control unit 20 described below. The term "radiation dose rate" denotes a radiation dose per unit time.

Specifically, in order to accurately detect the current value of the charged particle beam R with which the target 7 is irradiated, the current monitor 5 is provided immediately in front of the scanning electromagnet 6 on a downstream side from the quadrupole electromagnets 4 (a downstream side of the charged particle beam R) so as to eliminate the influence caused by the quadrupole electromagnets 4. In other words, since the scanning electromagnet 6 performs scanning so as to not irradiate the same place of the target 7 with the charged particle beam R at all times, in a case where the current monitor 5 is arranged on the downstream side from the scanning electromagnet 6, a large-sized current monitor 5 is necessary. In contrast, the current monitor 5 can be reduced in size by providing the current monitor 5 on an upstream side from the scanning electromagnet 6.

The scanning electromagnet 6 scans the charged particle beam R, thereby controlling irradiation of the charged particle beam R with respect to the target 7. The scanning electromagnet 6 controls an irradiation position of the charged particle beam R with respect to the target 7.

The neutron capture therapy device 1 generates the neutron beam N by irradiating the target 7 with the charged particle beam R, thereby emitting the neutron beam N heading toward the patient 50. The neutron capture therapy device 1 includes the target 7, a beam blockage body 9, the moderator 8, the collimator 10, and a gamma ray detection unit 11.

In addition, the neutron capture therapy device 1 includes the control unit 20. The control unit 20 is configured to include a central processing unit [CPU], a read-only memory [ROM], a random access memory [RAM], and the like. The control unit 20 is an electronic control unit which generally controls the neutron capture therapy device 1.

The target 7 generates the neutron beam N upon reception of irradiation which is performed with the charged particle beam R. In this case, the target 7 is formed of beryllium (Be), lithium (Li), tantalum (Ta), or tungsten (W), for example, and exhibits a circular plate shape having a diameter of 160 mm, for example.

The moderator 8 moderates energy of the neutron beam N generated in the target 7. The moderator 8 has a layered structure configured to include a first moderator 8A which mainly moderates fast neutrons included in the neutron beam N, and a second moderator 8B which mainly moderates epithermal neutrons included in the neutron beam N.

The beam blockage body 9 blocks the generated neutron beam N, a gamma ray which is generated in response to the generation of the neutron beam N, and the like so as not to allow the beam and the ray to be radiated to the outside. The beam blockage body 9 is provided so as to surround the moderator 8. An upper portion and a lower portion of the beam blockage body 9 extend from the moderator 8 to the upstream side of the charged particle beam R, and the gamma ray detection unit 11 is provided in each of the extension portions thereof.

The collimator 10 performs shaping of the irradiation field of the neutron beam N and has an opening 10a through which the neutron beam N passes. For example, the collimator 10 is a block-shaped member having the opening 10a at the center.

The gamma ray detection unit 11 detects a gamma ray generated from the neutron beam generation section M in response to the irradiation of the charged particle beam R in real time. As the gamma ray detection unit 11, it is possible to employ various gamma ray detection instruments such as a scintillator, an ionization chamber, and others. In the present embodiment, the gamma ray detection unit 11 is provided on the upstream side of the charged particle beam R from the moderator 8 on the periphery of the target 7.

The gamma ray detection unit 11 is disposed on each of the inner sides of the upper portion and the lower portion of the beam blockage body 9 which extends to the upstream side of the charged particle beam R. The number of gamma ray detection units 11 may be one or may be three or more, without being particularly limited. When providing three or more gamma ray detection units 11, the gamma ray detection units 11 can be provided at predetermined intervals so as to surround the outer circumference of the target 7. The gamma ray detection unit 11 outputs a detection result of a gamma ray to the control unit 20. The gamma ray detection unit 11 corresponds to the gamma ray detection unit disclosed in Claims.

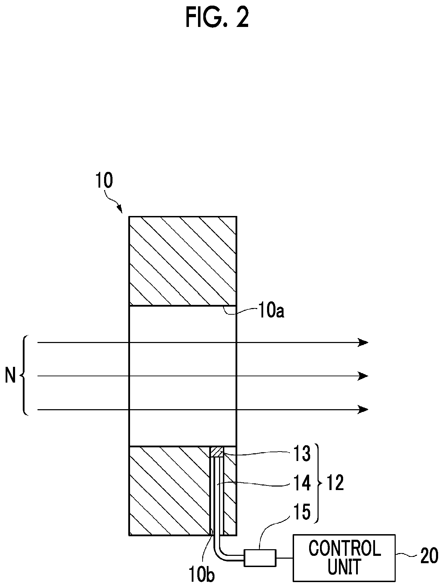

FIG. 2 is a cross-sectional diagram illustrating a neutron beam detector 12 which is provided inside the collimator 10. As illustrated in FIG. 2, inside the collimator 10, there is provided the neutron beam detector 12 for detecting the neutron beam N which passes through the opening 10a of the collimator 10 in real time. At least a portion of the neutron beam detector 12 is provided in a penetration hole 10b (a penetration hole formed in a direction orthogonal to the opening 10a) formed in the collimator 10. The neutron beam detector 12 includes a scintillator 13, a light guide 14, and an optical detector 15, for example.

The scintillator 13 is a phosphor which converts an incident neutron beam N into light. The internal crystal of the scintillator 13 is in an excitation state in accordance with the radiation dose of the incident neutron beam N, thereby generating scintillation light. The scintillator 13 is provided inside the penetration hole 10b of the collimator 10 and is exposed to the opening 10a of the collimator 10. The scintillator 13 emits light as the neutron beam N inside the opening 10a is incident on the scintillator 13. The scintillator 13 corresponds to the neutron beam detection section disclosed in Claims.

The light guide 14 is a member which transfers light generated in the scintillator 13. For example, the light guide 14 is configured to be formed with a flexible optical fiber bundle and the like. The optical detector 15 detects light which is transferred through the light guide 14. As the optical detector 15, for example, it is possible to employ various optical detection instruments such as a photo-multiplier and a phototube. The optical detector 15 outputs an electrical signal to the control unit 20 when light is detected.

The neutron beam detector 12 does not necessarily use the scintillator 13, and various neutron beam detection instruments such as the ionization chamber and others may be employed.

The control unit 20 includes a radiation dose calculation section (neutron beam dose calculation means) 21, an irradiation control section (control means) 22, and an error determination section (determination means) 23.

The radiation dose calculation section 21 measures (calculates) the radiation dose of the charged particle beam R in real time during the irradiation based on the detection result of the current monitor 5, the gamma ray detection unit 11, and the neutron beam detector 12.

Specifically, the radiation dose calculation section 21 measures (calculates) the radiation dose of the charged particle beam R with which the target 7 is irradiated, in real time based on the detection result of the current value of the charged particle beam R obtained by the current monitor 5. The radiation dose calculation section 21 performs iterated integration regarding time with the measured current value of the charged particle beam R, thereby calculating the radiation dose of the charged particle beam R in real time.

In addition, the radiation dose calculation section 21 measures (calculates) the radiation dose of a gamma ray in real time based on the detection result of the gamma ray obtained by the gamma ray detection unit 11. The radiation dose calculation section 21 configures the gamma ray dose measurement means disclosed in Claims, in association with the gamma ray detection unit 11.

Similarly, the radiation dose calculation section 21 measures (calculates) the radiation dose of the neutron beam N passing through the opening 10a of the collimator 10, based on the detection result of the neutron beam N obtained by the neutron beam detector 12. The radiation dose calculation section 21 configures the neutron beam dose measurement means disclosed in Claims, in association with the neutron beam detector 12.

The radiation dose calculation section 21 generally calculates the radiation dose of the neutron beam N generated in the target 7, in real time based on the radiation dose of the charged particle beam R, the radiation dose of the gamma ray, and the radiation dose of the neutron beam N which are calculated. In addition, the radiation dose calculation section 21 may correct the calculation result of the radiation dose of the neutron beam N based on the radiation dose of the charged particle beam R and the radiation dose of the gamma ray which are calculated, thereby calculating the radiation dose of the neutron beam N which is more reliable.

The irradiation control section 22 controls the irradiation of the charged particle beam R with respect to the target 7 based on the radiation dose of the neutron beam N which is calculated by the radiation dose calculation section 21. The irradiation control section 22 controls the irradiation of the charged particle beam R with respect to the target 7, thereby controlling the irradiation of the neutron beam N generated from the target 7, with respect to a patient. The irradiation control section 22 controls the irradiation of the neutron beam N so as to cause the radiation dose of the neutron beam N calculated by the radiation dose calculation section 21 to comply with the treatment plan which is set in advance.

The error determination section 23 determines whether generation of the neutron beam N in the target 7 is normal or abnormal, based on the radiation dose of the charged particle beam R, the radiation dose of the gamma ray, and the radiation dose of the neutron beam N which are calculated by the radiation dose calculation section 21.

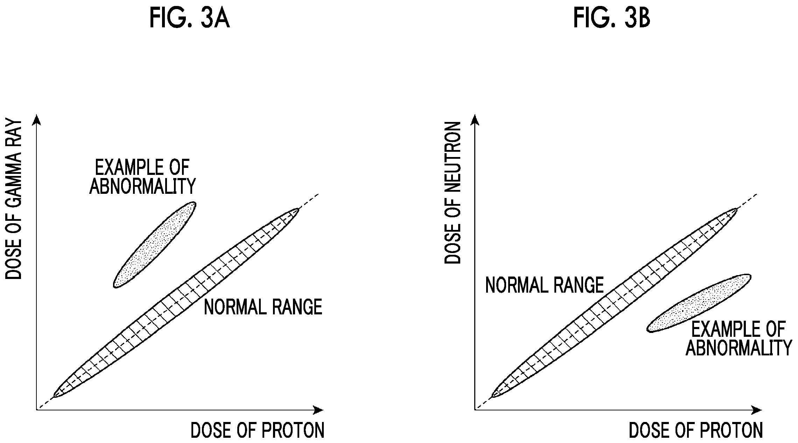

Here, FIG. 3(a) is a diagram for illustrating abnormality determination conducted based on a relationship between a dose of gamma rays and a dose of protons. The vertical axis in FIG. 3(a) indicates the dose of the gamma ray, and the horizontal axis in FIG. 3(a) indicates the dose of protons (corresponding to the radiation dose of the charged particle beam R). In addition, FIG. 3(b) is a diagram for illustrating abnormality determination conducted based on a relationship between a dose of neutrons and a dose of protons. The vertical axis in FIG. 3(b) indicates the dose of the neutron beam, and the horizontal axis in FIG. 3(b) indicates the dose of protons.

As illustrated in FIG. 3(a), there is a correlationship between the radiation dose of the charged particle beam R with which the target 7 of the neutron beam generation section M is irradiated, and the radiation dose of the gamma ray which is generated in the neutron beam generation section M. Therefore, when an abnormality occurs in generation of the neutron beam N, it is conceivable that an abnormality has occurred in the relationship therebetween. Accordingly, it is possible to determine whether generation of the neutron beam N is normal or abnormal based on the relationship between the dose of gamma rays and the dose of protons which are calculated.

Similarly in the relationship between the dose of neutrons and the dose of protons illustrated in FIG. 3(b) as well, when an abnormality occurs in generation of the neutron beam N, it is conceivable that an abnormality occurs in the relationship therebetween. Therefore, it is possible to determine whether generation of the neutron beam N is normal or abnormal. In FIGS. 3(a) and 3(b), the normal range and the example of abnormality in each of the relationships are illustrated.

A correlationship exists between the examples of abnormality illustrated in FIGS. 3(a) and 3(b). For example, when an abnormality occurs in scanning performed with the charged particle beam R performed by the scanning electromagnet 6, there is a concern that the beam duct 3 between the scanning electromagnet 6 and the target 7 is irradiated with the charged particle beam R instead of a proper position of the target 7. While the beam duct 3 is irradiated with the charged particle beam R, the neutron beam N is unlikely to be generated. In addition, since the radiation dose of the neutron beam N decreases and a large quantity of the gamma rays is generated in the beam duct 3 between the scanning electromagnet 6 and the target 7, the radiation dose of the gamma ray increases. Therefore, as illustrated in FIGS. 3(a) and 3(b), there is an occurrence of an abnormality in which the radiation dose of the gamma ray increases and the radiation dose of the neutron beam N decreases. The normal ranges and the examples of abnormality in FIGS. 3(a) and 3(b) are merely images which are schematically illustrated, and the contents of the present invention are by no means limited.

In addition, the error determination section 23 may determine whether generation of the neutron beam N is normal or abnormal directly based on the detection results of the current monitor 5, the gamma ray detection unit 11, and the neutron beam detector 12, instead of the calculation result of the radiation dose calculation section 21. For example, it is possible for the error determination section 23 to determine whether generation of the neutron beam N is normal or abnormal by storing the data map related to the relationship among each of the detection results in the normal range in advance, and contrasting each of the detection results and the data map.

The error determination section 23 stops irradiating (emission of the charged particle beam R from the cyclotron 2) the target 7 with the charged particle beam R when it is determined that generation of the neutron beam N is abnormal.

Subsequently, an operational effect of the neutron capture therapy device 1 according to the aforementioned first embodiment will be described.

In the neutron capture therapy device 1 according to the first embodiment, since the current value of the charged particle beam R for irradiating the target 7 is detected, and the gamma ray generated together with the neutron beam N in response to the irradiation of the charged particle beam R with respect to the target 7 is detected, it is possible to detect both the irradiation condition of the charged particle beam R before generating a neutron beam N and the generation condition of the gamma ray after the neutron beam N is generated. Therefore, it is possible to improve irradiation accuracy of the neutron beam N.

In other words, if only the current value of the charged particle beam R is measured as in the case of the related art, when there is an occurrence of an abnormality in which irradiation of the charged particle beam R is not performed with respect to a proper position of the target 7, for example, even though the measured current value of the charged particle beam R is normal, only the radiation dose of the neutron beam N lower than the radiation dose which is essentially obtainable is obtained. In contrast, according to the neutron capture therapy device 1, the abnormality can be detected by measuring the radiation dose of the gamma ray generated in the neutron beam generation section M in response to the irradiation of the charged particle beam R. Therefore, in the neutron capture therapy device 1, different from the case of the related art, when the above-described abnormality is detected, it is possible to adjust the irradiation (the irradiation position or the radiation dose) of the charged particle beam R or to stop the irradiation of the charged particle beam R. Thus, it is possible to improve irradiation accuracy of the neutron beam N.

In addition, according to the neutron capture therapy device 1, since the radiation dose of the neutron beam N heading toward the patient 50 is calculated based on the detection result of the current monitor 5 and the gamma ray detection unit 11, it is possible to determine whether or not the radiation dose of the neutron beam N complies with the treatment plan, thereby being advantageous to improve irradiation accuracy of the neutron beam.

In addition, according to the neutron capture therapy device 1, since the irradiation of the charged particle beam R with respect to the target 7 is controlled based on the calculation result of the radiation dose of the neutron beam N obtained by the radiation dose calculation section 21, the irradiation of the neutron beam N can be controlled so as to obtain the radiation dose which complies with the treatment plan. Therefore, it is possible to improve the irradiation accuracy of the neutron beam with which a patient is irradiated.

In addition, according to the neutron capture therapy device 1, since it is determined whether generation of the neutron beam N is normal or abnormal based on the detection result of the current monitor 5 and the detection result of the gamma ray detection unit 11, it is possible to detect an abnormality which occurs in generation of the neutron beam N such as an excessive radiation dose of the gamma ray compared to the radiation dose of irradiation of the charged particle beam R.

Moreover, according to the neutron capture therapy device 1, since the irradiation of the charged particle beam R with respect to the target 7 stops when it is determined that generation of the neutron beam N is abnormal, it is possible to prevent the patient 50 from being irradiated with the neutron beam N at the radiation dose which does not comply with the treatment plan.

In addition, according to the neutron capture therapy device 1, since the gamma ray detection unit 11 is provided on the upstream side of the charged particle beam R from the moderator 8 on the periphery of the target 7, it is possible to detect the gamma ray generated from the target 7 before the gamma ray is blocked by other members, thereby contributing to improvement of measurement accuracy of the radiation dose of the gamma ray. In addition, maintenance of the gamma ray detection unit 11 is also easily performed.

In addition, according to the neutron capture therapy device 1, since the scintillator 13 which detects the neutron beam N is provided inside the collimator 10 and is exposed to the opening 10a of the collimator 10 through which the neutron beam N passes, it is possible to detect the neutron beam N heading toward the patient 50 without blocking the traveling path of the neutron beam N. Moreover, according to the neutron capture therapy device 1, since the radiation dose of the neutron beam N is calculated in consideration of the detection result of the neutron beam detector 12 including the scintillator 13, compared to a case of including no neutron beam detector 12, it is possible to accurately measure the radiation dose of the neutron beam N, and thus, irradiation accuracy of the neutron beam N can be improved. In addition, according to the neutron capture therapy device 1, the detection result of the neutron beam detector 12 is also taken into consideration. Therefore, it is possible to more accurately determine the presence and the absence of an abnormality in generation of the neutron beam N.

### [Second Embodiment]

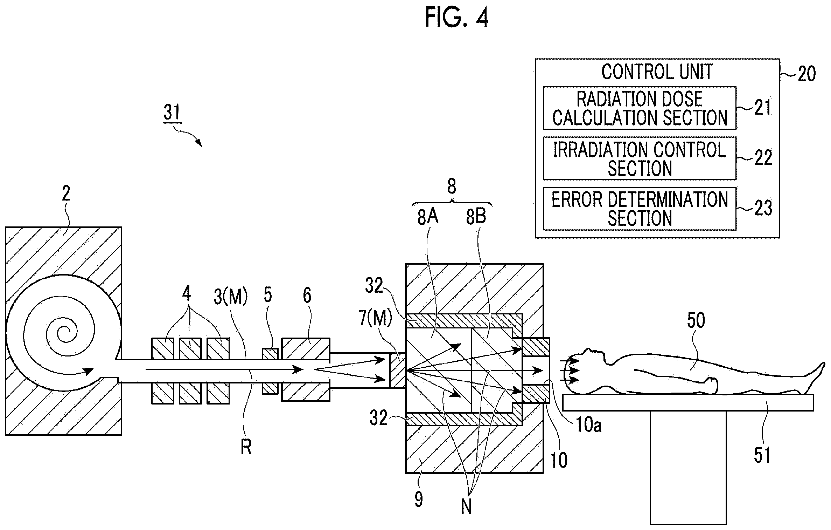

FIG. 4 is a diagram illustrating a neutron capture therapy device 31 according to a second embodiment. As illustrated in FIG. 4, the only difference in the neutron capture therapy device 31 according to the second embodiment compared to the first embodiment is a gamma ray detection unit 32.

Specifically, in the neutron capture therapy device 31 according to the second embodiment, the gamma ray detection unit 32 is provided between the moderator 8 and the beam blockage body 9. The gamma ray detection unit 32 is provided so as to surround the periphery of the moderator 8 (the first moderator 8A and the second moderator 8B), and the beam blockage body 9 surrounds the outer side thereof. The downstream side (the downstream side of the neutron beam N) of the gamma ray detection unit 32 makes a detour around the downstream side of the second moderator 8B, thereby reaching the vicinity of the collimator 10.

In the neutron capture therapy device 31 according to the second embodiment described above, it is possible to obtain the effect similar to that in the first embodiment. In addition, according to the neutron capture therapy device 31, since the gamma ray detection unit 32 is provided between the moderator 8 in which the neutron beam N travels and the beam blockage body 9 which surrounds the moderator 8, it is possible to capture the gamma ray in the forward direction of the neutron beam N, and thus, it is possible to detect a gamma ray which is advantageous to measure the neutron beam N. The forward direction of the neutron beam N denotes a main forward direction of the neutron beam N. The main forward direction of the neutron beam N is the forward direction of the charged particle beam R when irradiating the target 7 (precisely, the forward direction of the charged particle beam R when the scanning electromagnet 6 scans nothing).

In addition, in the neutron capture therapy device 31, since the gamma ray detection unit 32 is provided between the moderator 8 and the beam blockage body 9, the gamma ray can be prevented from being missed in detection, and thus, it is possible to prevent an abnormal state from being missed in detection.

Hereinbefore, favorable embodiments of the present invention are described. However, the present invention is not limited to the above-described embodiments. For example, the neutron beam detection section does not necessarily include the scintillator, and the ionization chamber may take the place thereof. In addition, the neutron capture therapy device does not necessarily provide the neutron beam detection section which detects the neutron beam.

In addition, the position and the shape of the gamma ray detection unit are not limited to those described above. For example, there is no need to provide the gamma ray detection unit in the overall space between the moderator and the beam blockage body. The gamma ray detection unit may be provided in only a portion therebetween. For example, the gamma ray detection unit may be disposed in a depression or the like provided between the first moderator and the beam blockage body. In this case, the number of the gamma ray detection units may be one or multiple. In a case of multiple units, the gamma ray detection units can be provided at predetermined intervals so as to surround the moderator. In addition, the gamma ray detection unit may be provided only between the second moderator and the beam blockage body, or may be provided in only the vicinity of the collimator.

In addition, the neutron capture therapy device according to an aspect of the present invention does not need to determine whether generation of a neutron beam is normal or abnormal based on the detection result of the current value of the charged particle beam and the detection result of the gamma ray. For example, the neutron capture therapy device may adopt a form in which a monitor of the control unit displays the detection result of the current value of the charged particle beam, the detection result of the gamma ray, and the detection result of the neutron beam so as to allow a worker or the like to determine whether the detection results are normal or abnormal.

In addition, the neutron capture therapy device according to an aspect of the present invention does not necessarily calculate the radiation dose of the neutron beam based on the detection result of the current value of the charged particle beam and the detection result of the gamma ray. The detection results may be utilized for only abnormality determination. Otherwise, the detection result of the current value of the charged particle beam and the detection result of the gamma ray may be utilized for only calculating the radiation dose of the neutron beam.

### Industrial Applicability

According to an aspect of the present invention, it is possible to provide a neutron capture therapy device in which irradiation accuracy of a neutron beam can be improved.

### Reference Signs List

1, 31 ... NEUTRON CAPTURE THERAPY DEVICE; 2 ... CYCLOTRON (ACCELERATOR) ; 3 ... BEAM DUCT; 4 ... QUADRUPOLE ELECTROMAGNET; 5 ... CURRENT MONITOR (CURRENT VALUE DETECTION MEANS); 6 ... SCANNING ELECTROMAGNET; 7 ... TARGET; 8 ... MODERATOR; 8A ... FIRST MODERATOR; 8B ... SECOND MODERATOR; 9 ... BEAM BLOCKAGE BODY; 10 ... COLLIMATOR; 10a ... OPENING; 10b ... PENETRATION HOLE; 11, 32 ... GAMMA RAY DETECTION UNIT (GAMMA RAY DETECTION UNIT, GAMMA RAY DOSE MEASUREMENT MEANS); 12 ... NEUTRON BEAM DETECTOR; 13 ... SCINTILLATOR (NEUTRON BEAM DETECTION SECTION, NEUTRON BEAM DOSE MEASUREMENT MEANS) ; 14 ... LIGHT GUIDE; 15 ... OPTICAL DETECTOR; 20 ... CONTROL UNIT; 21 ... RADIATION DOSE CALCULATION SECTION (NEUTRON BEAM DOSE CALCULATION MEANS, GAMMA RAY DOSE MEASUREMENT MEANS, NEUTRON BEAM DOSE MEASUREMENT MEANS) ; 22 ... IRRADIATION CONTROL SECTION (CONTROL MEANS) ; 23 ... ERROR DETERMINATION SECTION (DETERMINATION MEANS); 50 ... PATIENT (IRRADIATION TARGET) ; N ... NEUTRON BEAM; M ... NEUTRON BEAM GENERATION SECTION; R ... CHARGED PARTICLE BEAM

## Claims

1. A neutron capture therapy device (1, 31) configured to irradiate an irradiation target (7) with a neutron beam (N), the device comprising:
an accelerator (2) configured to emit a charged particle beam (R);
a neutron beam generation section (M) configured to include a target (7) which generates the neutron beam (N) when being irradiated with the charged particle beam (R);
current value detection means (5) for detecting a current value of the charged particle beam (R) with which the target (7) is irradiated;
gamma ray dose measurement means (11, 32) for measuring a radiation dose of a gamma ray which is generated from the neutron beam generation section (M) in response to irradiation of the charged particle beam,
**characterized by**
neutron beam dose calculation means (21) for calculating a radiation dose of the neutron beam (N) which is generated in the neutron beam generation section (M), based on a detection result of the current value detection means (5) and a measurement result of the gamma ray dose measurement means (11, 32).

2. The neutron capture therapy device (1, 31) according to Claim 1, further comprising:
control means (22) for controlling irradiation of the charged particle beam (R) with respect to the target (7) based on a calculation result of the neutron beam dose calculation means (21).

3. The neutron capture therapy device (1, 31) according to any one of Claims 1 to 2, further comprising:
determination means (23) for determining whether generation of the neutron beam (N) is normal or abnormal, based on the detection result of the current value detection means (5) and the measurement result of the gamma ray dose measurement means (11, 32).

4. The neutron capture therapy device (1, 31) according to Claim 3,
wherein the determination means (23) is configured to stop irradiating the target (7) with the charged particle beam (R) when it is determined that generation of the neutron beam (N) is abnormal.

5. The neutron capture therapy device (1) according to any one of Claims 1 to 4, further comprising:
a moderator (8) configured to moderate the neutron beam (N) which is generated in the neutron beam generation section; and
a beam blockage body (9) configured to be provided so as to surround the moderator (8),
wherein the gamma ray dose measurement means (11) includes a gamma ray detection unit (11) configured to detect the gamma ray, and
wherein the gamma ray detection unit (11) is provided on an upstream side of the charged particle beam (R) from the moderator (8) on the periphery of the target.

6. The neutron capture therapy device (31) according to any one of Claims 1 to 4, further comprising:
a moderator (8) configured to moderate the neutron beam (N) which is generated in the neutron beam generation section; and
a beam blockage body (9) configured to be provided so as to surround the moderator,
wherein the gamma ray dose measurement means (32) includes a gamma ray detection unit (32) configured to detect the gamma ray, and
wherein the gamma ray detection unit (32) is provided between the moderator (8) and the beam blockage body.

7. The neutron capture therapy device (1, 31) according to any one of Claims 1 to 6, further comprising:
a collimator (10) configured to include an opening (10a) for shaping an irradiation field of the neutron beam (N); and
neutron beam dose measurement means (12) for measuring the radiation dose of the neutron beam,
wherein the neutron beam dose measurement means includes a neutron beam detection section (13) configured to detect the neutron beam, and
wherein the neutron beam detection section (13) is provided inside the collimator and is exposed to the opening (10a).

## Patentansprüche

1. Neutroneneinfangtherapie-Vorrichtung (1,31), die so konfiguriert ist, dass sie ein Bestrahlungstarget (7) mit einem Neutronenstrahl (N) bestrahlt, wobei die Vorrichtung umfasst:
einen Beschleuniger (2), der so konfiguriert ist, dass er einen Strahl (R) geladener Teilchen ausstrahlt,
einen Neutronenstrahl-Erzeugerabschnitt (M), der so konfiguriert ist, dass er ein Target (8) beinhaltet, welches den Neutronenstrahl (N) erzeugt, wenn es mit dem Strahl (R) geladener Teilchen bestrahlt wird,
Stromwert-Detektionsmittel (5) zum Detektieren eines Stromwerts des Strahls (R) geladener Teilchen, mit dem das Target (7) bestrahlt wird,
Gammastrahlen-Dosis-Messmittel (11, 32) zum Messen einer Strahlungsdosis einer Gammastrahlung, die von dem Neutronenstrahl-Erzeugerabschnitt (M) in Reaktion auf die Bestrahlung durch den Strahl geladener Teilchen erzeugt wird,
**gekennzeichnet durch**
Neutronenstrahldosis-Berechnungsmittel (21) zum Berechnen einer Strahlungsdosis des Neutronenstrahls (N), der in dem Neutronenstrahl-Erzeugerabschnitt (M) erzeugt wird, basierend auf einem Detektionsergebnis der Stromwert-Detektionsmittel (5) und eines Messergebnisses der Gammastrahlen-Dosis-Messmittel (11, 32).

2. Neutroneneinfangtherapie-Vorrichtung (1, 31) nach Anspruch 1, weiter umfassend Steuerungsmittel (22) zum Steuern der Bestrahlung des Strahls (R) geladener Teilchen bezüglich des Targets (7), basierend auf einem Berechnungsergebnis des Neutronenstrahldosis-Berechnungsmittels (21).

3. Neutroneneinfangtherapie-Vorrichtung (1, 31) nach einem der Ansprüche 1 oder 2, weiter umfassend Bestimmungsmittel (23) zum Bestimmen, ob die Erzeugung des Neutronenstrahls (N) normal oder nicht normal ist, basierend auf dem Detektionsergebnis des Stromwert-Detektionsmittels (5) und des Messergebnisses der Gammastrahlendosis-Messmittel (11, 32).

4. Neutroneneinfangtherapie-Vorrichtung (1, 31) nach Anspruch 3, wobei das Bestimmungsmittel (23) so konfiguriert ist, dass es aufhört, das Target (7) mit dem Strahl (R) geladener Teilchen zu bestrahlen, wenn bestimmt ist, dass die Erzeugung des Neutronenstrahls (N) nicht normal ist.

5. Neutroneneinfangtherapie-Vorrichtung (1, 31) nach einem der Ansprüche 1 bis 4, weiter umfassend einen Moderator (8), der so konfiguriert ist, dass er den Neutronenstrahl (N), der in dem Neutronenstrahl-Erzeugungsabschnitt erzeugt wird, moderiert, und
einen Strahlblockierkörper (9), der so konfiguriert ist, dass er so angeordnet ist, dass er den Moderator (8) umgibt,
wobei das Gammastrahlen-Dosis-Messmittel (11) eine Gammastrahlen-Detektionseinheit (11) umfasst, die so konfiguriert ist, dass sie die Gammastrahlung detektiert, und
worin die Gammastrahlen-Detektionseinheit (11) an einer in Strahlrichtung gesehen vor dem Moderator (8) liegenden Seite des Strahls (R) geladener Teilchen in der Nähe des Targets vorgesehen ist.

6. Neutroneneinfangtherapie-Vorrichtung (1, 31) nach einem der Ansprüche 1 bis 4, weiter umfassend einen Moderator (8), der so konfiguriert ist, dass er den Neutronenstrahl (N), der in dem Neutronenstrahl-Erzeugungsabschnitt erzeugt wird, moderiert, und
einen Strahlblockierkörper (9), der so ausgestaltet ist, dass er so angeordnet ist, dass er den Moderator (8) umgibt,
wobei das Gammastrahlen-Dosis-Messmittel (32) eine Gammastrahlen-Detektionseinheit (32) umfasst, die so konfiguriert ist, dass sie die Gammastrahlung detektiert, und
worin die Gammastrahlen-Detektionseinheit (32) zwischen dem Moderator (8) und dem Strahlblockierkörper vorgesehen ist.

7. Neutroneneinfangtherapie-Vorrichtung (1, 31) nach einem der Ansprüche 1 bis 6, weiter umfassend einen Kollimator (10), der so konfiguriert ist, dass er eine Öffnung (10a) zum Formen eines Strahlungsfeldes des Neutronenstrahls (N) und Neutronenstrahl-Dosis-Messmittel (12) zum Messen der Strahlungsdosis des Neutronenstrahls umfasst,
wobei das Neutronenstrahl-Dosis-Messmittel einen Neutronenstrahl-Detektionsabschnitt (13) umfasst, der so konfiguriert ist, dass er den Neutronenstrahl detektiert und wobei der Neutronenstrahl-Detektionsabschnitt (13) innerhalb des Kollimators vorgesehen und der Öffnung (10a) ausgesetzt ist.

## Revendications

1. Dispositif de thérapie par capture de neutrons (1, 31) configuré pour irradier une cible d'irradiation (7) avec un faisceau de neutrons (N), le dispositif comprenant :
un accélérateur (2) configuré pour émettre un faisceau de particules chargées (R) ;
une section de génération de faisceau de neutrons (M) configurée pour comprendre une cible (7) qui génère le faisceau de neutrons (N) lorsqu'elle est irradiée avec le faisceau de particules chargées (R) ;
un moyen de détection de valeur actuelle (5) destiné à détecter une valeur actuelle du faisceau de particules chargées (R) avec lequel la cible (7) est irradiée ;
un moyen de mesure de dose de rayons gamma (11, 32) destiné à mesurer une dose de radiation de rayons gamma qui est généré par la section de génération de faisceau de neutrons (M) en réponse à l'irradiation du faisceau de particules chargées,
**caractérisé par**
un moyen de calcul de dose de faisceau de neutrons (21) destiné à calculer une dose de radiation du faisceau de neutrons (N) qui est généré dans la section de génération de faisceau de neutrons (M), sur la base d'un résultat de détection du moyen de détection de valeur actuelle (5) et d'un résultat de mesure du moyen de mesure de dose de rayons gamma (11, 32).

2. Dispositif de thérapie par capture de neutrons (1, 31) selon la revendication 1, comprenant en outre :
un moyen de commande (22) destiné à contrôler l'irradiation du faisceau de particules chargées (R) par rapport à la cible (7) sur la base d'un résultat de calcul du moyen de calcul de dose de faisceau de neutrons (21).

3. Dispositif de thérapie par capture de neutrons (1, 31) selon l'une quelconque des revendications 1 à 2, comprenant en outre :
un moyen de détermination (23) destiné à déterminer si la génération du faisceau de neutrons (N) est normale ou anormale, sur la base du résultat de détection du moyen de détection de valeur actuelle (5) et du résultat de mesure du moyen de mesure de dose de rayons gamma (11, 32).

4. Dispositif de thérapie par capture de neutrons (1, 31) selon la revendication 3,
dans lequel le moyen de détermination (23) est configuré pour arrêter l'irradiation de la cible (7) avec le faisceau de particules chargées (R) lorsqu'il est déterminé que la génération du faisceau de neutrons (N) est anormale.

5. Dispositif de thérapie par capture de neutrons (1) selon l'une quelconque des revendications 1 à 4, comprenant en outre :
un modérateur (8) configuré pour modérer le faisceau de neutrons (N) qui est généré par la section de génération de faisceau de neutrons ;
un corps de blocage de faisceau (9) configuré pour être prévu de façon à entourer le modérateur (8),
dans lequel le moyen de mesure de dose de rayons gamma (11) comprend une unité de détection de rayons gamma (11) configurée pour détecter les rayons gamma, et
dans lequel l'unité de détection de rayons gamma (11) est prévue sur un côté amont du faisceau de particules chargées (R) par rapport au modérateur (8), sur la périphérie de la cible.

6. Dispositif de thérapie par capture de neutrons (31) selon l'une quelconque des revendications 1 à 4, comprenant en outre :
un modérateur (8) configuré pour modérer le faisceau de neutrons (N) qui est généré dans la section de génération de faisceau de neutrons ;
et
un corps de blocage de faisceau (9) configuré pour être prévu de façon à entourer le modérateur,
dans lequel le moyen de mesure de dose de rayons gamma (32) comprend une unité de détection de rayons gamma (32) configurée pour détecter les rayons gamma, et
dans lequel l'unité de détection de rayons gamma (32) est prévue entre le modérateur (8) et le corps de blocage de faisceau.

7. Dispositif de thérapie par capture de neutrons (1, 31) selon l'une quelconque des revendications 1 à 6, comprenant en outre :
un collimateur (10) configuré pour comprendre une ouverture (10a) destinée à mettre en forme un champ d'irradiation du faisceau de neutrons (N) ; et
un moyen de mesure de dose de faisceau de neutrons (12) destiné à mesurer la dose de radiation du faisceau de neutrons,
dans lequel le moyen de mesure de dose de faisceau de neutrons comprend une section de détection de faisceau de neutrons (13) configurée pour détecter le faisceau de neutrons, et
dans lequel la section de détection de faisceau de neutrons (13) est prévue à l'intérieur du collimateur et est exposée à l'ouverture (10a).
